# EUROPÄISCHE PATENTSCHRIFT

(11) **EP 3 545 937 B1**
(45) Veröffentlichungstag und Bekanntmachung des Hinweises auf die Patenterteilung: **18.05.2022**
(21) Anmeldenummer: 18164596.1
(22) Anmeldetag: 28.03.2018
(51) Int. Cl.: A61J 3/07, A61K 9/48

(54) **KAPSELBUCHSE FÜR STECKKAPSELN UND BUCHSENSYSTEM**
CAPSULE BUSHING FOR PLUG CAPSULES AND BUSHING SYSTEM
CAPSULE POUR CUPULES POUR CACHETS ET SYSTÈME DE CAPSULES

(43) Veröffentlichungstag der Anmeldung: 02.10.2019
(73) Patentinhaber: Harro Höfliger Verpackungsmaschinen GmbH, 71573 Allmersbach im Tal (DE)
(72) Erfinder: Gall, Steffen, 71573 Allmersbach im Tal (DE); Müller, Daniel, 71573 Allmersbach im Tal (DE)
(74) Vertreter: Zurhorst, Stefan

(56) Entgegenhaltungen:
- WO-A1-2008/141861
- DE-A1-102010 038 544
- DE-U1-202011 003 680
- US-B1- 6 367 228

## Beschreibung

Die Erfindung betrifft eine Kapselbuchse einer Füllmaschine für Steckkapseln mit den Merkmalen nach dem Oberbegriff des Anspruchs 1, sowie ein Buchsensystem umfassend eine solche Kapselbuchse.

Insbesondere im Pharmabereich, aber auch im Bereich von Nahrungsergänzungsmitteln oder dgl. werden schluckbare Kapseln eingesetzt, in deren Innenraum Wirkstoffpräparate oder dgl. eingefüllt sind. Derartige Kapseln sind zweiteilig aus zwei Kapselhälften ausgeführt und bestehen aus einem Kapselunterteil sowie einem darauf aufgestecktem Kapseloberteil. Verbreitete Kapselmaterialien sind Hartgelatine, HPMC (Hydroxypropylmethylcellulose) oder dgl..

Leerkapseln werden für die Befüllung im lose zusammengesteckten Zustand angeliefert und einem Kapseltransportsystem zugeführt. Dieses umfasst ein Segmentsystem mit Kapselbuchsen, namentlich mit Kapseloberteilbuchsen und mit Kapselbuchsen, wobei die lose zusammengesteckten Leerkapseln zunächst in den Kapseloberteilbuchsen zu liegen kommen. Hiervon ausgehend wird das Kapselunterteil aus dem Kapseloberteil beispielsweise mittels Unterdruck herausgezogen und in einen Aufnahmeraum der Kapselbuchse eingeführt. In der Kapselbuchse findet die Befüllung des Kapselunterteils statt. Hieran anschließend wird das Kapselunterteil beispielsweise mittels eines Stempels zur Kapseloberteilaufhahme gedrückt und dort in das Kapseloberteil abschließend eingeführt.

Ein Umfangswandabschnitt des Aufnahmeraums dient einer losen Zentrierung der Kapselwand. Zu beachten ist hierbei, dass die einzelnen Kapselteile gewisse Streuungen beziehungsweise Toleranzen in ihren äußeren Abmaßen aufweisen. Außerdem kann es vorkommen, dass Kapseln mit zwar gleichen Nennmaßen, aber wegen unterschiedlicher Materialien mit unterschiedlichem mechanischem Verhalten verarbeitet werden sollen. Damit solche Kapselhälften frei in die jeweiligen Kapselbuchsen eingeführt und von dort auch wieder ausgestoßen werden können, ist im Stand der Technik eine entsprechend großzügige, spielbehaftete Dimensionierung des Aufnahmeraums insbesondere im Bereich seiner Umfangswand vorgesehen.

In einem solchen Szenario besteht ein kritischer Effekt in den sogenannten Kapselsprüngen. Die Kapselunterteile werden dabei zunächst ordnungsgemäß in den Aufnahmeraum der jeweiligen Kapselbuchsen eingeführt bzw. eingesaugt, was mit hoher Geschwindigkeit geschieht. Nach Beendigung des impulsartigen Unterdruckstoßes können die Kapselunterteile vom Boden abprallen und dabei den genannten Kapselsprung ausführen. Der Sprung eines Kapselunterteils kann auch Rückwirkungen auf das darüber befindliche Kapseloberteil haben, welches dann seinerseits zu einem Kapselsprung neigt. Im weniger kritischen Fall geht hierdurch Zeit verloren, bis sich alle Kapselhälften in ihrer Lage beruhigt haben und die Befüllung begonnen werden kann. Im kritischeren Fall finden einzelne Kapselhälften nicht mehr in ihre vorgesehene Position zurück oder springen sogar aus ihrer Buchse heraus. In der Folge kommt es zur Produktion von Ausschuss oder sogar zum Produktionsstillstand. Der genannte Effekt kann durch eine enger tolerierte Anpassung der Buchsengeometrie an die jeweils zu verarbeitenden Kapseltypen gemindert werden. Das setzt aber voraus, dass für jeden konkreten Kapseltyp ein angepasster Satz von Kapselbuchsen vorhanden ist. Eine universelle Einsetzbarkeit für verschiedene Kapseln innerhalb eines bestimmten Durchmesserbereichs geht damit verloren, wobei außerdem eine vollständige Vermeidung der Kapselsprünge nicht gelingt.

Aus der WO 2008/141861 A1 ist eine vergleichbare Kapselunterteilbuchse bekannt, in deren Aufnahmeraum ein Kapselunterteil zum Zwecke der Versiegelung gehalten ist. Der Aufnahmeraum ist nach unten offen, um einen Stößel von unten einführen zu können. Innenseitig weist der Halter einen nicht näher bezeichneten ringförmigen Absatz auf, der der Abstützung des halbkugeligen Kapselbodens bei fehlendem Stößel dient. Der genannte Absatz nimmt also die Funktion eines Bodenabschnittes des Aufnahmeraums wahr.

Der Erfindung liegt die Aufgabe zugrunde, eine gattungsgemäße Kapselbuchse derart weiterzubilden, dass ihre Betriebssicherheit gesteigert ist.

Diese Aufgabe wird durch eine Kapselbuchse mit den Merkmalen des Anspruchs 1 gelöst.

Der Erfindung liegt des Weiteren die Aufgabe zugrunde, ein Buchsensystem umfassend eine Kapselbuchse und eine Kapselhälfte derart aufeinander abzustimmen, dass ein leichtes Ein- und Ausfädeln und dennoch ein zuverlässiges Halten der Kapselhälfte in der Kapselbuchse erreicht ist.

Diese Aufgabe wird durch ein Buchsensystem mit den Merkmalen des Anspruchs 8 gelöst.

Nach der Erfindung ist eine Kapselbuchse vorgesehen, bei der der Aufnahmeraum zwischen dem Bodenabschnitt und dem Umfangswandabschnitt einen Klemmabschnitt aufweist, wobei der Klemmabschnitt einen kleineren freien Querschnitt als der Umfangswandabschnitt aufweist. In einem entsprechenden Buchsensystem, welches eine solche Kapselbuchse und eine passende Kapselhälfte umfasst, weist der Klemmabschnitt des Aufnahmeraums einen freien Klemmdurchmesser auf, welcher kleiner ist als der Kapselteildurchmesser der zugeordneten Kapselhälfte. Mit anderen Worten wird also nicht der gesamte Aufnahmeraum in seinem Querschnitt soweit verkleinert, dass die Kapselhälfte klemmend geführt und gehalten wird. Vielmehr sieht die Erfindung eine nur lokale Klemmung mittels des Klemmabschnittes vor. Hierdurch kann die Kapsel bzw. die Kapselhälfte frei eingeführt bzw. angesaugt werden, während die Klemmwirkung des Klemmabschnittes nur im Bereich der Endposition eintritt, in der die Kapselhälfte ordnungsgemäß positioniert ist. Umgekehrt reicht es nach dem Füllvorgang aus, das Kapselunterteil mit einem nur kurzen Stoß aus seiner Klemmung zu befreien, woran sich dann eine freie, unbehinderte Bewegung zur Einführung in das Kapseloberteil anschließen kann. Sinngemäß das Gleiche gilt auch für den dann nachfolgenden Ausstoß der fertig befüllten und zusammengesteckten Kapsel. Die örtlich im Klemmabschnitt begrenzte Klemmung stellt sicher, dass die jeweilige Kapselhälfte auch bei sehr schnell getakteten Zyklen und hoher Bewegungsgeschwindigkeit nicht wieder aus ihrer Zielposition zurückprallt. Vielmehr kann erreicht werden, dass die Kapselhälften bereits nach sehr kurzer Zeit ihre Füllposition eingenommen haben und für den sich anschließenden Füllvorgang auch beibehalten. Kapselsprünge sind zuverlässig vermieden, sodass die Betriebssicherheit deutlich gesteigert ist. Freie Beweglichkeit und lokal begrenzte Klemmwirkung funktionieren innerhalb einer beträchtlichen Bandbreite von Maßtoleranzen und mechanisch unterschiedlichen Verhaltensweisen verschiedener Kapseln, so dass eine spezifische Anpassung der Kapselbuchsen an bestimmte Kapseltypen kaum oder sogar gar nicht erforderlich ist.

In vorteilhafter Weiterbildung der Erfindung weist der Klemmabschnitt auch einen kleineren freien Querschnitt als der Bodenabschnitt auf. Hierbei wird eine besondere mechanische Eigenschaft des Kapselunterteils genutzt: In radialer Richtung ist der kalottenförmige Kapselboden steifer als die etwa zylindrische Kapselwand. Der Kapselboden wird also beim Einführen zunächst durch den verengten Klemmabschnitt gezwängt, kann sich dann aber im größeren Querschnitt des Bodenabschnitts wieder etwas aufweiten. Der Klemmabschnitt umgreift mit seinem kleineren freien Querschnitt nur noch die weichere, nachgiebigere Kapselwand. Hierdurch entsteht über eine reine Klemmung hinausgehend eine Verrastung nach dem Prinzip eines Druckknopfs. Es wird eine präzise definierte Lagepositionierung der Kapselhälfte erreicht, die nur über einen kleinen axialen Weg wirkt und deshalb die freie Beweglichkeit der Kapselhälfte beim Ein- oder Ausbringen praktisch nicht beeinträchtigt. Insbesondere grenzt der Klemmabschnitt unmittelbar an den Bodenabschnitt an. Dadurch ist die axiale Lage der Kapselhälfte eindeutig und praktisch spielfrei in beide Richtungen, also nach oben und nach unten definiert.

Es kann zweckmäßig sein, einzelne radiale Vorsprünge oder dergleichen auf der Innenwand der Aufnahmeöffnung vorzusehen, um hierdurch den Klemmabschnitt zu bilden. In einer bevorzugten Ausführungsform ist der Klemmabschnitt durch einen innenseitig der Aufnahmeöffnung umlaufenden Ringwulst gebildet und weist insbesondere einen gerundeten Querschnitt auf. Mit der Erzielung der gewünschten Klemm- bzw. Rastwirkung gehen hierbei nur sehr kleine örtliche Flächenpressungen einher, welche auf die Kapselwand wirken. Der gerundete Querschnitt erlaubt ein schädigungsfreies Ein- und Ausfädeln der Kapselhälfte.

In einer vorteilhaften Ausführungsform ist die Kapselbuchse eine Kapselunterteilbuchse zur Aufnahme einer als Kapselunterteil ausgebildeten Kapselhälfte. Hierdurch kann zuverlässig vermieden werden, dass das mit hoher Geschwindigkeit eintretende Kapselunterteil vom Bodenabschnitt zurückprallt. Alternativ oder zusätzlich kann es zweckmäßig sein, dass die Kapselbuchse eine Kapseloberteilbuchse zur Aufnahme einer als Kapseloberteil ausgebildeten Kapselhälfte ist. Rückwirkungen vom Kapselunterteil oder andere Einflüsse können nicht mehr dazu führen, dass das Kapseloberteil einen Sprung ausführt.

Ein Ausführungsbeispiel der Erfindung ist nachfolgend anhand der Zeichnung näher beschrieben. Es zeigen:
- Fig. 1: in einer Längsschnittdarstellung ein erfindungsgemäßes Buchsensystem mit einer Steckkapsel, mit einer Kapselunterteilbuchse und mit einer Kapseloberteilbuchse zur Handhabung von Kapseloberteil und Kapselunterteil beim Füllen,
- Fig. 2: in einer Längsschnittdarstellung die Kapselunterteilbuchse nach Fig. 1 mit Einzelheiten zu ihrer geometrischen Ausgestaltung,
- Fig. 3: in einer Längsschnittdarstellung die Kapseloberteilbuchse nach Fig. 1 mit Einzelheiten zu ihrer geometrischen Ausgestaltung, und
- Fig. 4: in einer vergrößerten Darstellung die in den Fig. 2 und 3 mit IV gekennzeichnete Einzelheit betreffend die Querschnittsform eines Ringwulstes zur Bildung des Klemmabschnittes.

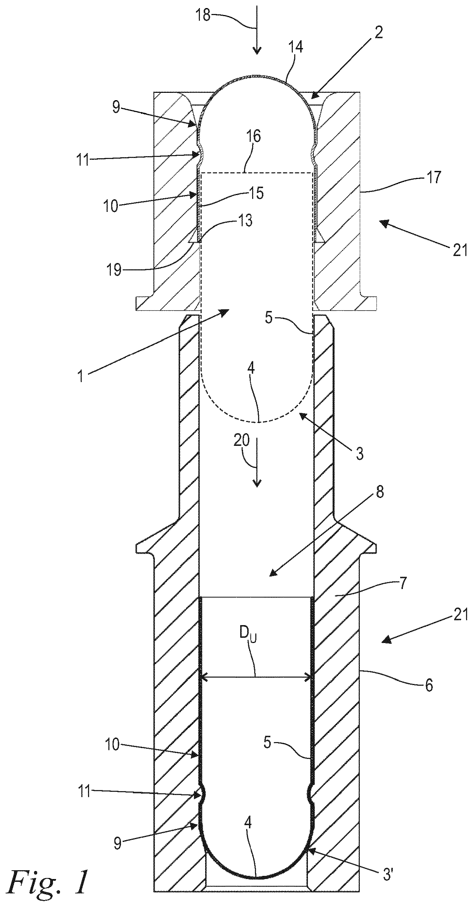

Fig. 1 zeigt in einer Längsschnittdarstellung ein erfindungsgemäßes Buchsensystem als Teil eines nicht näher dargestellten Transportsystems innerhalb einer Füllmaschine für Steckkapseln 1. Die Steckkapseln 1, von denen hier zur besseren Übersicht nur ein einziges Exemplar gezeigt ist, werden beispielsweise im Pharmabereich oder im Bereich von Nahrungsergänzungsmitteln eingesetzt, und enthalten im befüllten Zustand ein Wirkstoffpräparat. Im fertig befüllten und verschlossenen Zustand sind sie zum Verschlucken durch eine Person vorgesehen. Als Kapselmaterial kommen verschiedene Materialen wie Hartgelatine, HPMC oder dgl. in Betracht, welches sich nach dem Verschlucken auflöst und den Kapselinhalt freigibt.

Die zweiteilige Steckkapsel 1 ist aus zwei Kapselhälften, nämlich einem Kapseloberteil 2 und einem Kapselunterteil 3 zusammengesetzt, wobei das Kapselunterteil 3 in die offene Seite des Kapseloberteils 2 eingeschoben ist. Das Kapseloberteil 2 besteht in üblicher Bauform aus einem halbkugeligen Kapselboden 14, an die sich eine obere Kapselwand 15 anschließt. An seiner offenen Seite ist das Kapseloberteil 2 durch eine umlaufende Kante 13 der oberen Kapselwand 15 begrenzt. Das Kapselunterteil 3 ist analog zum Kapseloberteil 2 aufgebaut und besteht aus einem halbkugeligen, kalottenförmigen Kapselboden 4, an den sich eine zylindrische untere Kapselwand 5 anschließt. An seiner dem Kapseloberteil 2 zugewandten und dort eingeführten Seite ist das Kapselunterteil 3 mit einer umlaufenden Kante 16 der zylindrischen Kapselwand 5 begrenzt, wobei im zusammengesteckten Zustand von Kapseloberteil 2 und Kapselunterteil 3 ein Teil der zylindrischen Kapselwand 5 samt ihrer umlaufenden Kante 16 innerhalb der oberen Kapselwand 15 des Kapseloberteils 2 zu liegen kommt.

Das erfindungsgemäße Buchsensystem umfasst zumindest eine der vorgenannten Kapselhälften und zumindest eine dieser Kapselhälfte zugeordnete, in nachstehend näher beschriebener Weise geometrisch auf die Kapselhälfte abgestimmte Kapselbuchse 21. Nach der Erfindung kann dies eine Kapselunterteilbuchse 6 und eine als Kapselunterteil 3 ausgebildete Kapselhälfte oder eine Kapseloberteilbuchse 17 und eine als Kapseloberteil 2 ausgebildete Kapselhälfte sein. Im gezeigten bevorzugten Ausführungsbeispiel umfasst das erfindungsgemäße Buchsensystem je ein Kapselunterteil 3, ein Kapseloberteil 2 sowie zwei zugeordnete Kapselbuchsen 21, nämlich eine als Kapselunterteilbuchse 6 ausgebildete Kapselbuchse 21 zur Aufnahme des Kapselunterteils 3 und eine als Kapseloberteilbuchse 17 ausgebildete Kapselbuchse 21 zur Aufnahme des Kapseloberteils 2. Im praktischen Betrieb sind mehrere solcher Buchsensysteme gleichzeitig bzw. synchron im Einsatz.

Leere Steckkapseln 1 werden in einem Zustand angeliefert, bei denen die Kapselunterteile 3 in jeweils zugeordnete Kapseloberteile 2 lose eingesteckt sind. In diesem Zustand wird eine einzelne Kapsel 1 in die Kapseloberteilbuchse 17 von oben entsprechend einem Pfeil 18 eingeführt. Die als Kapseloberteilbuchse 17 ausgeführte obere Kapselbuchse 21 umfasst einen Grundkörper 7 mit einem darin ausgeformten Aufnahmeraum 8 für das Kapseloberteil 2, wobei dieser obere Aufnahmeraum 8 einen dem Kapselboden 14 des Kapseloberteils 2 zugeordneten Bodenabschnitt 9 und einen Umfangswandabschnitt 10 zur Zentrierung der Kapselwand 15 aufweist. Der Aufnahmeraum 8 ist Teil einer gestuften Durchgangsbohrung, innerhalb derer sich an den Aufnahmeraum 8 ein ringförmiger Auflageabsatz 19 anschließt. Die umlaufende Kante 13 des Kapseloberteils 2 liegt im eingeführten Zustand auf einem Auflageabsatz 19 auf.

Unterhalb der Kapseloberteilbuchse 17 ist die Kapselunterteilbuchse 6 koaxial dazu positioniert und umfasst ebenfalls einen Grundkörper 7 mit einem darin ausgebildeten Aufnahmeraum 8 für eine Kapselhälfte, hier für das Kapselunterteil 3. Auch der untere Aufnahmeraum 8 umfasst einen Umfangswandabschnitt 10 und einen hier allerdings darunter befindlichen Bodenabschnitt 9, wobei dieser Bodenabschnitt 9 dem Kapselboden 4 des Kapselunterteils 3 zugeordnet ist, und wobei der Umfangswandabschnitt 10 der Zentrierung der Kapselwand 5 des Kapselunterteils 3 dient.

Beispielsweise mittels Vakuum oder dgl. wird das Kapselunterteil 3 aus dem Kapseloberteil 2 herausgezogen und in den Aufnahmeraum 8 der Kapselunterteilaufnahme 6 entsprechend einem Pfeil 20 eingesaugt, bis es in seiner mit 3' bezeichneten Endposition mit seinem nach unten weisenden Kapselboden 4 auf dem gegenüber dem Umfangswandabschnitt 10 im Durchmesser verringerten Bodenabschnitt 9 zu liegen kommt und im Ruhezustand dort in axialer Richtung nach unten abgestützt wird. Hierbei wird die Kapselwand 5 des Kapselunterteils 3 durch den Umfangswandabschnitt 10 des Aufnahmeraums 8 zentriert und in radialer Richtung gestützt.

Die Kapseloberteilbuchse 17 wird nun in nicht dargestellter Weise entfernt, so dass die Einheit aus Kapselunterteil 3 und Kapselunterteilbuchse 6 von oben frei zugänglich ist. Das Kapselunterteil 3 wird daraufhin mit dem nicht dargestellten Wirkstoffpräparat befüllt, während es in der Kapselunterteilbuchse 6 gehalten ist. Nach der Befüllung erfolgt ein Verschließen der Steckkapsel 1. Hierzu wird die Einheit aus Kapseloberteil 2 und Kapseloberteilbuchse 17 wieder zurück in die Position nach Fig. 1 verfahren, und das Kapselunterteil 3 wird beispielsweise mittels eines nicht dargestellten Stempels entgegen dem Pfeil 20 nach oben zurück in das Kapseloberteil 2 hineingedrückt.

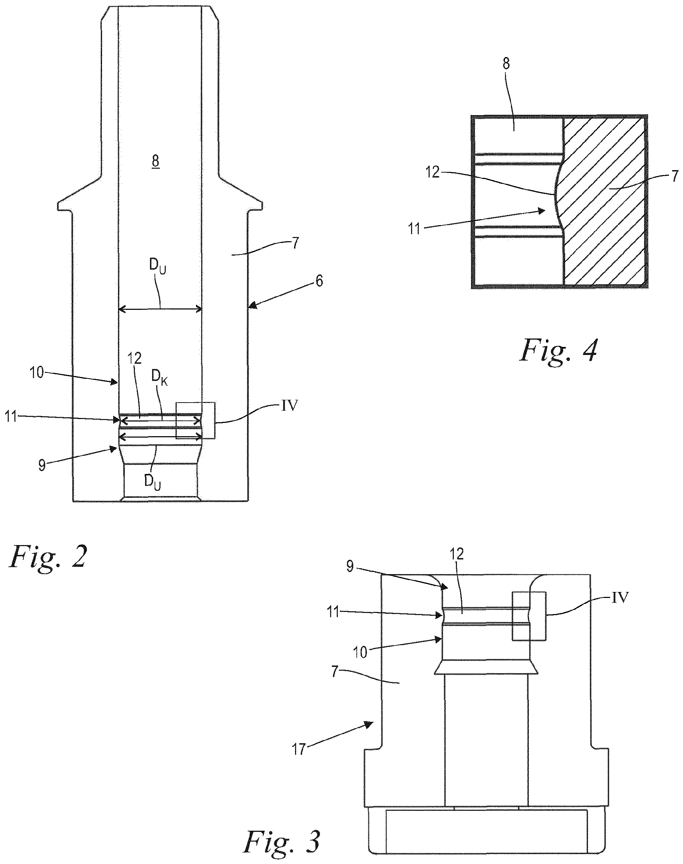

Fig. 2 zeigt in einer Längsschnittdarstellung die Kapselunterteilbuchse 6 nach Fig. 1. Aus der Zusammenschau mit Fig. 1 ist erkennbar, dass der im Grundkörper 7 koaxial dazu ausgebildete Aufnahmeraum 8 neben dem bereits erwähnten Bodenabschnitt 9 und dem ebenfalls erwähnten Umfangswandabschnitt 10 noch einen Klemmabschnitt 11 mit verringertem Querschnitt umfasst. Der Klemmabschnitt 11 befindet sich unmittelbar oberhalb des Bodenabschnittes 9, grenzt also unmittelbar an diesen nach oben an. Darüber befindet sich der Umfangswandabschnitt 10. Es kann aber auch zweckmäßig sein, dass sich zwischen dem Bodenabschnitt 9 und dem Klemmabschnitt 11 noch ein Teil des Umfangswandabschnittes 10 befindet. Jedenfalls ist die Geometrie des Aufnahmeraums 8 derart an die Geometrie des zum Einsatz kommenden Kapselunterteils 3 angepasst, dass der Bodenabschnitt 9 allein den halbkugeligen Kapselboden 4 aufnimmt, während der darüber befindliche Klemmabschnitt 11 unmittelbar oberhalb des Kapselbodens 4 mit der Kapselwand 5 des Kapselunterteils 6 in Wechselwirkung tritt.

Weiter ergibt sich aus der Zusammenschau der Fig. 1 und 2, dass der Klemmabschnitt 11 einen kleineren freien Querschnitt als der Umfangswandabschnitt 10 hat und optional auch einen kleineren freien Querschnitt als der Bodenabschnitt 9 aufweist. Im vorliegenden Fall ist dies durch einen bestimmten Klemmdurchmesser D_{K} des Klemmabschnitts 11 wie folgt erreicht: Das Kapselunterteil 3 weist gemäß Fig. 1 entlang seiner Kapselwand 5, zumindest aber im Übergang der Kapselwand 5 zum Kapselboden einen Kapselteildurchmesser Du auf. Für eine freie axiale Beweglichkeit des Kapselunterteils 3 innerhalb des Aufnahmeraums 8 ist der freie Querschnitt des Umfangswandabschnittes 10 hieran angepasst und weist ebenfalls den Kapselteildurchmesser Du auf, wobei etwas radiales Spiel zur Kapselwand 5 zweckmäßig sein kann. Das Gleiche gilt auch für den obersten Bereich des Bodenabschnitts 9. Jedenfalls weist der Klemmabschnitt 11 einen kleineren freien Querschnitt als der Umfangswandabschnitt 10 und auch als der oberste, an den Klemmabschnitt 11 angrenzende Bereich des Bodenabschnitts 9 auf, wozu im gezeigten Ausführungsbeispiel der freie Klemmdurchmesser D_{K} kleiner ist als der Kapselteildurchmesser Du.

Fig. 3 zeigt in einer Längsschnittdarstellung die Kapseloberteilbuchse 17 nach Fig. 1. Aus der Zusammenschau mit Fig. 2 ist erkennbar, dass die Kapseloberteilbuchse 17 nach Fig. 3 insoweit analog zur Kapselunterteilbuchse 6 nach Fig. 2 aufgebaut ist, als dass auch ihr Aufnahmeraum 8 zwischen dem Bodenabschnitt 9 und dem Umfangswandabschnitt 10 einen Klemmabschnitt 11 aufweist. Für die geometrische Ausgestaltung des Klemmabschnittes 11 insbesondere hinsichtlich seines hier nicht eingezeichneten Klemmdurchmessers und dessen Abstimmung auf den ebenfalls nicht eingezeichneten Kapselteildurchmesser des Kapseloberteils 2 (Fig. 1) gilt in analoger Weise das zur Kapselunterteilbuchse 6 nach Fig. 2 Gesagte.

In den Figuren 2 und 3 ist jeweils eine Einzelheit mit IV gekennzeichnet, welche vergrößert in Fig. 4 dargestellt ist. Demnach ist der Klemmabschnitt 11 durch einen innenseitig der Aufnahmeöffnung 8 umlaufenden Ringwulst 12 gebildet. Der Ringwulst 12 weist im gezeigten bevorzugten Ausführungsbeispiel einen gerundeten Querschnitt auf. Es kann aber auch ein Querschnitt mit Ecken, Rastkanten oder dergleichen zweckmäßig sein. Außerdem sind im Rahmen der Erfindung andere Ausführungen des Klemmabschnitts beispielsweise durch die Anordnung von radial nach innen über die Kontur des Umfangswandabschnitts 10 hervorstehenden Vorsprüngen möglich.

Jedenfalls wird durch die erfindungsgemäße Ausgestaltung erreicht, dass das Kapselunterteil 3 unmittelbar nach dem Herausziehen aus dem Kapseloberteil 2 (Fig. 1) beim Auftreffen auf den Bodenabschnitt 9 mittels des Klemmabschnittes 11 klemmend bzw. rastend gehalten wird und deshalb nicht beim Aufprall auf den Bodenabschnitt 9 von diesem zurückspringen kann (Kapselsprung). Hierzu drückt sich der Klemmabschnitt 11 in die Kapselwand 5 des Kapselunterteils 3 unmittelbar oberhalb dessen Kapselboden ein, wobei die Kapselwand radial nach innen unter elastischer Verformung leicht nachgibt. Es kann eine ungestörte Befüllung des Kapselunterteils 3 erfolgen. Umgekehrt reicht ein geringer Impuls von unten aus, um anschließend das befüllte Kapselunterteil 3 aus seiner Klemmung bzw. Rastung zu befreien und wieder zurück in das zugehörige Kapseloberteil 2 zu befördern. Sinngemäß das Gleiche gilt auch für das Kapseloberteil 2, welches rastend in der Kapseloberteilbuchse 17 gehalten und am Springen gehindert wird. Ein Hinausdrücken der komplettierten Steckkapsel 1 ist dennoch mittels eines nur geringen Stoßes oder Impulses möglich.

## Patentansprüche

1. Kapselbuchse (21) einer Füllmaschine für Steckkapseln (1), wobei die Stecckapseln (1) zwei Kapselhälften mit jeweils einem Kapselboden (4, 14) und mit einer Kapselwand (5, 15) aufweisen, wobei die Kapselbuchse (21) einen Grundkörper (7) mit einem darin ausgeformten Aufnahmeraum (8) für die Kapselhälfte umfasst, und wobei der Aufnahmeraum (8) einen dem jeweiligen Kapselboden (4, 14) zugeordneten Bodenabschnitt (9) und einen Umfangswandabschnitt (10) zur Zentrierung der Kapselwand (5, 15) aufweist,
**dadurch gekennzeichnet, dass** der Aufnahmeraum (8) zwischen dem Bodenabschnitt (9) und dem Umfangswandabschnitt (10) einen Klemmabschnitt (11) aufweist, wobei der Klemmabschnitt (11) einen kleineren freien Querschnitt als der Umfangswandabschnitt (10) aufweist.

2. Kapselbuchse nach Anspruch 1,
**dadurch gekennzeichnet, dass** der Klemmabschnitt (11) einen kleineren freien Querschnitt als der Bodenabschnitt (9) aufweist.

3. Kapselbuchse nach Anspruch 1 oder 2,
**dadurch gekennzeichnet, dass** der Klemmabschnitt (11) unmittelbar an den Bodenabschnitt (9) angrenzt.

4. Kapselbuchse nach einem der Ansprüche 1 bis 3,
**dadurch gekennzeichnet, dass** der Klemmabschnitt (11) durch einen innenseitig der Aufnahmeöffnung (8) umlaufenden Ringwulst (12) gebildet ist.

5. Kapselbuchse nach einem der Ansprüche 1 bis 4,
**dadurch gekennzeichnet, dass** der Ringwulst (12) einen gerundeten Querschnitt aufweist.

6. Kapselbuchse nach einem der Ansprüche 1 bis 5,
**dadurch gekennzeichnet, dass** die Kapselbuchse (21) eine Kapselunterteilbuchse (6) zur Aufnahme einer als Kapselunterteil (3) ausgebildeten Kapselhälfte ist.

7. Kapselbuchse nach einem der Ansprüche 1 bis 6,
**dadurch gekennzeichnet, dass** die Kapselbuchse (21) eine Kapseloberteilbuchse (17) zur Aufnahme einer als Kapseloberteil (2) ausgebildeten Kapselhälfte ist.

8. Buchsensystem umfassend eine Kapselbuchse (21) nach einem der Ansprüche 1 bis 7, wobei eine Kapselhälfte im Ubergangsbereich von ihrem Kapselboden (4) zu ihrer Kapselwand (5) einen Kapselteildurchmesser (Du) aufweist,
**dadurch gekennzeichnet, dass** der Klemmabschnitt (11) einen freien Klemmdurchmesser (D_{K}) aufweist, welcher kleiner ist als der Kapselteildurchmesser (Du).

## Claims

1. Capsule socket (21) of a filling machine for two-piece capsules (1), said two-piece capsules (1) having two capsule halves, each with a capsule base (4, 14) and with a capsule wall (5, 15), the capsule socket (21) comprising a main body (7) and, formed in the latter, a receiving space (8) for the capsule half, and the receiving space (8) having a base portion (9), assigned to the respective capsule base (4, 14), and a circumferential wall portion (10) for centering the capsule wall (5, 15),
**characterized in that** the receiving space (8) has a clamping portion (11) between the base portion (9) and the circumferential wall portion (10), the clamping portion (11) having a smaller free cross section than the circumferential wall portion (10).

2. Capsule socket according to Claim 1,
**characterized in that** the clamping portion (11) has a smaller free cross section than the base portion (9).

3. Capsule socket according to Claim 1 or 2,
**characterized in that** the clamping portion (11) directly adjoins the base portion (9).

4. Capsule socket according to one of Claims 1 to 3,
**characterized in that** the clamping portion (11) is formed by an annular bead (12) on the inside face of the receiving opening (8).

5. Capsule socket according to one of Claims 1 to 4,
**characterized in that** the annular bead (12) has a rounded cross section.

6. Capsule socket according to one of Claims 1 to 5,
**characterized in that** the capsule socket (21) is a capsule bottom socket (6) for receiving a capsule half configured as a capsule bottom (3).

7. Capsule socket according to one of Claims 1 to 6,
**characterized in that** the capsule socket (21) is a capsule top socket (17) for receiving a capsule half configured as a capsule top (2).

8. Socket system comprising a capsule socket (21) according to one of Claims 1 to 7, a capsule half having a capsule part diameter (D_{U}) in the transition region from its capsule base (4) to its capsule wall (5),
**characterized in that** the clamping portion (11) has a free clamping diameter (D_{K}), which is smaller than the capsule part diameter (Du).

## Revendications

1. Douille de capsule (21) d'une machine de remplissage pour capsules emboîtables (1), les capsules emboîtables (1) présentant deux moitiés de capsule ayant respectivement un fond de capsule (4, 14) et ayant une paroi de capsule (5, 15), la douille de capsule (21) comprenant un corps de base (7) avec un espace de réception (8) formé dans celui-ci pour les moitiés de capsule, et l'espace de réception (8) présentant une section de fond (9) associée au fond de capsule respectif (4, 14) et une section de paroi périphérique (10) pour le centrage de la paroi de capsule (5, 15),
**caractérisée en ce que** l'espace de réception (8) présente une section de serrage (11) entre la section de fond (9) et la section de paroi périphérique (10), la section de serrage (11) présentant une section transversale libre plus petite que la section de paroi périphérique (10).

2. Douille de capsule selon la revendication 1, **caractérisée en ce que** la section de serrage (11) présente une section transversale libre plus petite que la section de fond (9).

3. Douille de capsule selon la revendication 1 ou 2, **caractérisée en ce que** la section de serrage (11) est directement adjacente à la section de fond (9).

4. Douille de capsule selon l'une quelconque des revendications 1 à 3, **caractérisée en ce que** la section de serrage (11) est formée par un bourrelet annulaire (12) entourant l'ouverture de réception (8) sur le côté intérieur.

5. Douille de capsule selon l'une quelconque des revendications 1 à 4, **caractérisée en ce que** le bourrelet annulaire (12) présente une section transversale arrondie.

6. Douille de capsule selon l'une quelconque des revendications 1 à 5, **caractérisée en ce que** la douille de capsule (21) est une douille de partie inférieure de capsule (6) pour la réception d'une moitié de capsule configurée en tant que partie inférieure de capsule (3).

7. Douille de capsule selon l'une quelconque des revendications 1 à 6, **caractérisée en ce que** la douille de capsule (21) est une douille de partie supérieure de capsule (17) pour la réception d'une moitié de capsule configurée en tant que partie supérieure de capsule (2).

8. Système de douille comprenant une douille de capsule (21) selon l'une quelconque des revendications 1 à 7, une moitié de capsule présentant un diamètre de partie de capsule (D_{U}) dans la zone de transition entre son fond de capsule (4) et sa paroi de capsule (5),
**caractérisé en ce que** la section de serrage (11) présente un diamètre de serrage libre (Dₖ) qui est plus petit que le diamètre de partie de capsule (Dᵤ).
